# EUROPEAN PATENT APPLICATION

(11) **EP 3 895 761 A1**
(43) Date of publication of application: **20.10.2021**
(21) Application number: 21168813.0
(22) Date of filing: 16.04.2021
(51) Int. Cl.: A61P 13/00, A61K 36/45, A61K 36/82

(54) **CRANBERRY EXTRACT FOR PREVENTING AND TREATING UNCOMPLICATED LOWER URINARY TRACT INFECTIONS**

(30) Priority: 16.04.2020 IT 202000008095
(71) Applicant: Unifarco S.p.A., 32035 Santa Giustina (BL) (IT)
(72) Inventor: Baratto, Giovanni, 32035 Santa Giustina BL (IT)
(74) Representative: Perani & Partners S.p.A.

(57) **Abstract**

The present invention relates to a cranberry extract in which the degree of polymerization (%m/m) in total proanthocyanidins (PACs) is between 12.5 and 15%, and in which the ratio (weight of all proanthocyanidin polymers) /(total weight of proanthocyanidins) is > 60%. This cranberry extract is used as a single active ingredient or as an adjuvant for the prevention and treatment of uncomplicated lower urinary tract infections.

## Description

### FIELD OF THE INVENTION

The present invention relates to a cranberry extract for use as an active ingredient or adjuvant in the treatment and prevention of uncomplicated lower urinary tract infections.

### BACKGROUND ART

Uncomplicated urinary tract infections affect approximately 150 million people worldwide each year, and in the United States, for example, the annual social cost for this issue amounts to more than 3.5 billion dollars. Uncomplicated urinary tract infections (or UTIs) comprise lower urinary tract infections (cystitis, urethritis, prostatitis). Urinary tract infections which occur in a morphologically and functionally undamaged urinary system and in the absence of specific comorbidities are defined as "uncomplicated". In particular, cystitis is defined as acute or chronic inflammation of the bladder or urethra usually caused by an infection following bacterial colonization or, more rarely, by trauma or external agents (e.g., radiation therapy). The most common symptoms are: dysuria, pollakiuria, nocturia, stranguria, macrohaematuria, suprapubic pain, urgency incontinence, hyperchromic and foul-smelling urine. The medical history of underlying risk factors increases the likelihood of diagnosing UTIs. Interestingly, a woman who complains of classic lower urinary tract symptoms, in the absence of vaginal irritation or purulent secretions, has about 95% chance of having a UTI (1).

UTIs have different prevalence rates depending on several variables, but especially in relation to gender and age. In the United States, it is estimated that 25-40% of women between the age of 20 and 40 have had at least one episode of UTI. Similarly, an estimated 20% of adult women in Europe experience at least one episode of UTI during their lifetime. In addition, the different incidences in the two sexes are evident: only 0.0006-0.0008 UTI/person per year in the male population, compared to 0.5-0.7 UTI/person per year for females of the same age (2). The incidence of UTIs also appears to be age-related. In the first years of life, males have the highest incidence of UTIs, while during childhood, the incidence is 2% (with females 10 times more at risk than males). At school age, the incidence is 5% in females and very low in males. The incidence in women tends to increase with age and during periods of intense sexual activity. In menopause, women are at increased risk for frequent changes in the urinary tract, endocrine system and the onset of comorbidities (1).

As mentioned above, UTIs are for the most part caused by the colonization of pathogenic strains in the urogenital epithelium. Specifically, the microorganisms responsible for the infection are commonly of enteric origin. The main uropathogenic strains of *E. coli* (so-called UPEC) derive from the faecal intestinal microbiota and are distinguished by their ability to adhere, grow, resist host defences and colonize the urinary tract. UPEC strains are responsible for more than 85% of outpatient UTIs and 50% of nosocomial infections, although *Staphylococcus saprophyticus, Proteus mirabilis, Klebsiella pneumoniae* and *Enterococcus faecalis* are also among the aetiological agents of the disease. UPEC-dependent UTIs are mainly nosocomial (1). UPEC strains reach the urinary tract mainly due to the anatomical continuity between perineal/perianal and vaginal areas (3). Therefore, UTIs originate when the uropathogenic agent residing in the intestine contaminates the periurethral area, subsequently colonizing the urethra. From here, it migrates into the bladder and expresses piles and adhesins resulting in colonization and invasion of the surface of the "umbrella cells" (so called for the convex shape of the membrane facing the lumen). Accordingly, in order to counteract the action of the extracellular bacteria, the inflammatory response manifested by neutrophil infiltration in the affected site is triggered. However, more virulent strains can elude the immune system by modifying their morphology and/or invading host cells. For example, one of said bacterial defence mechanisms is "bacterial camouflage", which allows the pathogen to resist the action of neutrophils as well as multiply and form biofilms. Therefore, the production of toxins and proteases follows, which induce cell damage and the release of nutrients essential for bacterial survival and to reach the kidneys. The colonization of the latter culminates in the in situ production of bacterial toxins, again causing tissue damage. If the bacteria are able to cross the epithelial barrier of the renal tubules, untreated UTIs may progress and evolve into bacteraemia (3). In addition, in subjects with a weakened immune system (due to diseases or pharmacological treatments) or altered intestinal barrier permeability, the likelihood of bacterial translocation from the intestinal lumen to the underlying tissues increases until circulating in the blood and, therefore, the peripheral districts. More specifically, most bacteria that cause septicaemia following translocation from the intestinal tract belong to the species E. *coli* and are referred to as ExPEC ("extraintestinal pathogenic E. coli"). There may be a loss of barrier integrity underlying the translocation which manifests through thinning of the mucin layer and/or reduction of tight junction protein expression. E. *coli* themselves can increase wall permeability by expressing enzymes called "chip away", such as the SsIE protein capable of degrading mucin and α-hemolysin. The latter belongs to the group of lipases capable of generating permeability outbreaks (small holes in the epithelium) that allow the paracellular passage of the microorganism through the intestinal wall. In addition, some ExPECs have the ability to invade the lamina propria by transcytosing the cells of the intestinal immune system (dendritic cells or M cells) to reach the basolateral side of the mucosa (4), (5).

The primary condition for the onset of a UTI is the adhesion of pathogenic bacteria on the urogenital epithelial cells. The bacterial adhesion is mediated by structures of the bacterial cell surface called adhesins which allow adhesion to host cell membranes. These adhesins constitute the virulence factors of the pathogen. Specifically, fimbriae (particularly type P and type I fimbriae) and membrane proteins (haemagglutinins) are recognized. The former originate from the membrane of the bacterium and the free end thereof has adhesion molecules called lectins. Bacterial lectins are capable of binding characteristic sugars, such as D-mannose and L-fucosium, covalently bound to proteins and constituting glycolipids present on the cytoplasmic membrane of the bladder parietal cells and the intestinal epithelial cells of the host. The adhesion is followed by bacterial colonization and the induction of tissue damage (6). Further, the UPEC strains, for example, are capable of binding the protein Tamm-Horsfall (or uromodulin) which is abundantly present in urine. The ExPEC strains (or extraintestinal pathogenic E. coli) are also capable of adhering to and invading the intestinal mucosa and above all of moving to peripheral organs by virtue of the expression and consequent use of piles whose key protein is FimH. Finally, additional virulence factors include: capsular polysaccharides, hemolysines, cytotoxic necrotizing factor (CNF-1), aerobactins, biofilm proteins (antigen 43), protectins, iron uptake receptors and O antigen (1), (4).

From a diagnostic point of view, in the presence of clinical symptoms, the presence of any pathogen in the urine is evaluated. Laboratory analyses allow the detection and identification of the pathogen through the urine culture examination of the midstream urine samples. This method also allows the detection of the bacteriuria level. It should be noted that the minimum level of demonstrable bacteriuria is not standardized and well defined in the literature. As a result, laboratories set the cut-off for diagnosis at 10⁵ CFU/ml, although it is applied only and exclusively in cases of asymptomatic bacteriuria and on subjects with a bladder catheter. In many cases, the cut-off can be reduced to 10³ CFU/ml for specific pathogens and clinical situations such as symptomatic and male infections. In more sensitive cases, such as frequent, severe or refractory relapses to therapy and/or cases where suspicion of underlying uropathy is stronger, more in-depth investigations such as urodynamic study, urinary cystography and cystoscopy can be included. However, most outpatient UTIs are uncomplicated (1).

Fortunately, our body is able to react through endogenous defence systems. In fact, multiple defence mechanisms are available, in urine and more generally in the urinary tract. These include the continuous and unidirectional flow of urine which promotes the cleaning of the surfaces of the urinary tract; mucosal immunity mediated by secretory IgAs which can reduce the adhesiveness of bacterial cells and their invasive capacity; secretion of chemokines (IL-6 and IL-8) which exert a protective action; pH, urea concentration and urine osmolarity; organic acids which significantly limit the proliferation of pathogens. In addition, under physiological conditions, the urothelium represents a compact barrier by virtue of the presence of intercellular junctions and membrane proteins such as uroplachins and a urothelial coating composed of a layer of proteoglycans and glycosaminoglycans (GAGs). Equally important are bacteriokines (α and β defensins) and catelicidines, both with bactericidal properties. By virtue of the multiple defence mechanisms, the urogenital system is therefore protected from the attack of potentially pathogenic agents.

At a time when such endogenous defences are no longer sufficient to counteract the pathogen on their own, it is necessary to act externally by treating, for example, the pathogenic infection with specific antibiotics. The main problem of the use of antibiotics is the development of multi-resistance thereto by the bacteria responsible for this type of infections. Some clusters of intracellular bacteria can persist even months without being affected by the immune system and antibiotics (1). In addition to resistance, other particularly virulent strains are able to "internalize" into mucosal cells, forming quiescent and persistent reservoirs which cannot be attacked by antibiotics and are capable of re-triggering relapsed infections or asymptomatic bacteriuria. This has been demonstrated by *in vivo* studies and would explain the pathogenesis of recurrent cystitis and in particular those in which the same responsible bacterial strain is always detected. Evidence of this pattern has also been recorded in the human bladder. These types of UTIs are fairly recurrent manifestations in the population and are often difficult to treat. At least 30% of women with a first episode of UTI experience subsequent infection within 3-4 months. A recurrent UTI is such when at least two episodes (recorded with culture examination) occur in 6 months or at least three within a year. The incidence of recurrent UTIs is higher in menopausal women and is considered recurrent if caused by the same uropathogen within 30 days of the previous episode or reinfections if caused by different pathogenic strains. The UPEC strains responsible for recurrent UTIs have multiple virulence factors that make them particularly aggressive (1).

Preventive treatment with active ingredients which do not generate or promote resistance phenomena could contribute to reducing the often non-performing use of antibiotics (2). There are substances in nature which are capable of both preventing and combating the pathogenic action in the urinary tract.

For example, cranberry (*Vaccinium macrocarpon Ait.*)*,* commonly known as "American cranberry", is a small plant native to the north-east of the United States which produces red berries. Cranberry is known for its complex and abundant phytochemical composition characterized by the presence especially of flavan-3-ols, proanthocyanidins (or PACs), anthocyanins, benzoic acid and ursolic acid.

Flavan-3-ols are molecules characterized by a chemical structure with 15 carbon atoms like all flavonoids, consisting of two phenyl rings (A and B) joined by a heterocyclic 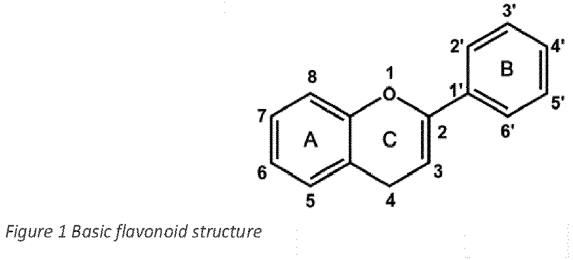 ring called C.

However, flavan-3-ols simultaneously have differences, such as the absence of the C2-C3 double bond and the presence of some hydroxyls.

Depending on the configuration of the OH group in position C3 and the benzene ring (B) in C2, the flavonols are defined as catechins (*trans* configuration) or epicatechins (*cis* configuration); four different isomers can thus be described

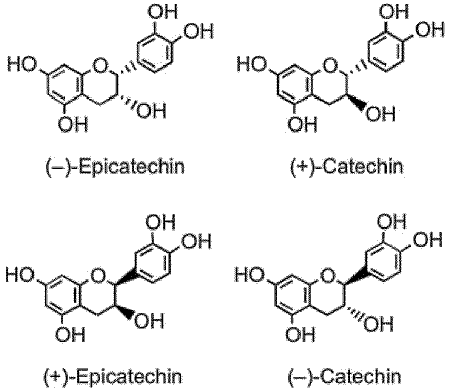

The oligomers and polymers of cranberry flavan-3-ols are called proanthocyanidins (PACs or also called condensed tannins). PACs include a heterogeneous group of chemical structures which differ in their constituent units, binding types and degree of polymerization. (-)-Epicatechin is the predominant constituent unit of the cranberry PACs; (+)-catechin and (epi)gallocatechins are present only in traces.

The structure of proanthocyanidins depends mainly on the inter-flavonol bonds: the variation of these bonds involves a classification into type A and type B 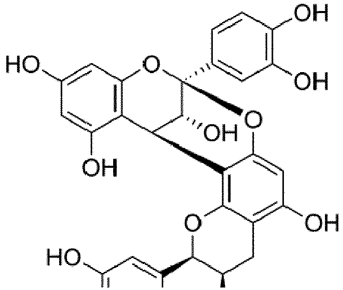 proanthocyanidins. In type A, the monomers are linked by C2-O-C5 or C2-O-C7 bond, while in type B, the monomers are linked by C4-C6 or C4-C8 bond

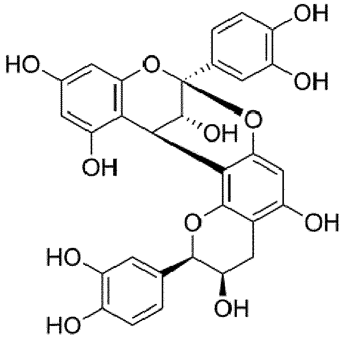

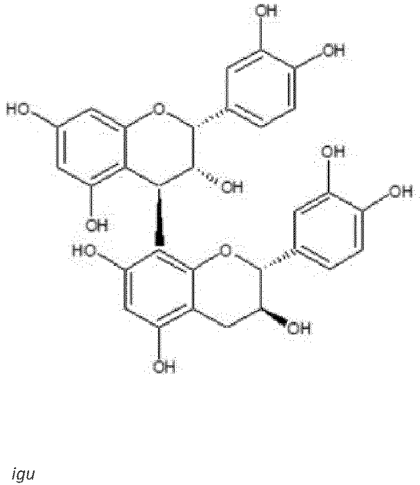

The position difference of the inter-flavanol bond and the constituent units further offers structural diversity to the higher oligomers; thus, the number of isomers increases along with the degree of polymerization.

The antibacterial activity of cranberry has been attributed to its ability to acidify urine for years. However, we know that the urinary pH remains unchanged after taking therapeutic doses. Today, cranberry is known to act by inhibiting bacterial adhesion, promoted essentially by type I and II fimbriae, to the mucosa of the bladder, a decisive phase for the development of urinary tract infections (7).

PAC-As are considered the most important active compounds of cranberry due to their anti-adhesive properties against fimbriated bacteria, both those susceptible to antibiotics and antibiotic-resistant ones (8). Furthermore, cranberry appears to act by inhibiting the formation of the bacterial biofilm (9). The anti-adhesive activity of cranberry has been demonstrated *in vivo* in animals and humans (10), (11). Among the phytotherapies used, cranberry and the extracts thereof are the only ones indicated by the guidelines of the European Urology Association. The best results were recorded in clinical trials where cranberry was used in the prophylaxis of urinary tract infections and not for curative purposes. In a 2002 clinical study involving 150 sexually active women aged 21 to 72 years, there was a reduction in antibiotic use and a statistically significant reduction in the number of patients with at least one urinary tract infection during the year. In 2008, the results of an interesting meta-analysis of 10 clinical trials were published. The conclusions of this work highlighted the effectiveness of cranberry in significantly reducing the incidence of urinary tract infections (9). The recommended dosage for the management of urinary infections and in particular for the reduction of bacteriuria and pyuria is 300 ml of cranberry juice containing 36 mg of PAC as measured by the BL-DMAC method (12), (13), (14). However, in the acute phase, dosages greater than or equal to 72 mg/day of PAC are recommended (12).

Low molecular PACs are believed to be effectively absorbed by the intestine and, through blood circulation, reach the urinary tract where they exert their anti-adhesive action (12), (16), (17), (18), (19). On the other hand, still others argue that the effect of the PACs is indirect, i.e., based on the catabolites of the PACs by the intestinal microbiota. Especially for high molecular weight PACs, the phenolic compounds (e.g., simple phenols, benzoic and phenylacetic acids, phenylpropionic acid) which derive from the metabolism thereof are consequently more easily absorbable at the intestinal level, reaching the urinary tract with the antibacterial effect (20), (21) while another part is excreted with faeces (20). This was confirmed by the study conducted with high molecular weight PACs extracted from Salicone (22). An *in vitro* study (23) showed the ability of high molecular weight PACs (extracted from apple and cedar) to inhibit the microbiota-mediated catabolism thereof. Further, other evidence from *in vitro* studies (24) shows the ability of high molecular weight PACs, containing a high number of type A bonds, to agglutinate an ExPEC strain (isolated from women with recurrent UTIs) preventing adhesion, colonization and therefore also translocation. In addition, PACs are capable of modulating immune activity. An *in vivo* study (25) conducted in 2014 demonstrated the ability of PACs to interact with lymphoid tissue associated with the intestinal mucosa (or GALT), promoting the activity thereof and production of mucosal IgAs. Furthermore, cocoa, grape and pine procyanidins are agonists (promoters of expansion) of Tγδ lymphocytes, a subpopulation of mucosal T cells responsible for modulating the intestinal immune response and epithelial repair (26). The importance of the molecular weight of polyphenols in the regulation of immune response can also be seen from another study conducted on inflamed colon cells (Caco-2 and HT-29 human colon cancer cells). High polymerization procyanidins derived from cocoa were more effective in counteracting colonocyte inflammation, and in particular, preserving membrane integrity and reducing the expression of inflammatory cytokines such as IL-8 (27). Finally, numerous *in vitro* and *in vivo* animal model studies have shown that the consumption of foods rich in polyphenols could positively affect barrier integrity by acting directly on the expression of proteins which form the narrow junctions. Therefore, it is possible to state that polyphenols contribute to the maintenance of intestinal barrier homoeostasis. Green tea flavonols, for example, increase the expression of *Akkermansia,* a bacterium involved in maintaining barrier functions as it is capable of preserving mucus layer thickness. Bilberry anthocyanins promote the production of short chain fatty acids (SCFAs) by promoting the growth of *Lactobacillus* and *Bifidobacterium* and inhibiting potentially harmful species such as *Verrucomicrobia* and *Euryarchaeota.* The same berries can reduce TNF-α and IL-6 levels in the colon, contributing to the restoration of intestinal barrier integrity (28).

Both direct and indirect models are likely to be valid and the effect of cranberry is the result of the synergistic action of PAC and the metabolites thereof. Furthermore, the intestinal absorption of PACs tends to decrease with the increasing degree of polymerization thereof (17), (18).

### SUMMARY OF THE INVENTION

The applicant has now surprisingly found a cranberry extract which despite containing proanthocyanidins having a high degree of polymerization, is effective in the treatment and prevention of uncomplicated lower urinary tract infections.

This cranberry extract is characterized by the degree of polymerization (%m/m) in total proanthocyanidins (PACs) between 12.5 and 15%, preferably between 13 and 14.5%, and in which the ratio (weight of all proanthocyanidin polymers) /(total weight of proanthocyanidins) is > 60%, preferably between 70 and 90%. Such an extract can be used as a single active ingredient or as an adjuvant for use in the treatment and prevention of uncomplicated lower urinary tract infections.

### DETAILED DESCRIPTION

For the purposes of the present invention, the term "comprising" or "containing" is not intended to exclude the presence of further components not listed after such a definition, while the term "consisting" or "constituted" is meant to exclude further components not listed after such a definition.

Extract or plant extract means a preparation based on plants or derivatives thereof (leaves, seeds, etc.) which are subject to extraction and component concentration processes.

For the purpose of the present invention, polymer means oligomers such as dimers, trimers, tetramers, pentamers, hexamers.

Degree of polymerization or %m/m means the percent content by weight of the single monomeric or oligomeric PAC detected by the HPLC-FLD method, which will be described below, on the total weight of the extract.

The degree of polymerization of the PACs is therefore the sum of the degree of polymerization of the single monomeric PAC and of all the oligomeric PACs.

Relative degree of polymerization percentage means the weight percentage of the single monomeric or oligomeric PAC on the total weight of all the PACs.

Adjuvant substance means a substance which, when combined with the drug used for a specific treatment of a disease, increases or supports the pharmacological action of said drug, or if administered subsequently during the period of suspension of said drug, prevents the recurrence of said disease.

For the purposes of the present invention, "uncomplicated urinary tract infections" (or UTIs) are lower urinary tract infections (cystitis, urethritis, prostatitis) while "uncomplicated" means urinary tract infections which are occur in a morphologically and functionally undamaged urinary system and in the absence of specific comorbidities.

Preferably, in said extract for use, the total proanthocyanidins consist of:
- type A proanthocyanidins (PAC-A), condensed together through the C2-O-C5 or C2-O-C7 bond, and
- type B proanthocyanidins (PAC-B), condensed together through the C4-C6 or C4-C8 bond.

According to the present invention, the weight ratio of PAC-A polymers to PAC-B polymers is preferably between 1.5 and 3, preferably between 1.8 and 2.6.

For the purpose of the present invention, the PAC-A to PAC-B ratio (or PAC-A/PAC-B) is a relevant weight aspect. In fact, without wishing to be bound by any scientific theory, it is hypothesized that the high molecular weight PAC-As contained in the cranberry extract according to the present invention exert a complementary and synergistic action to the low molecular weight PAC-As in combating bacterial urinary infections by acting on three levels:
- reducing proinflammatory cytokines
- promoting intestinal barrier integrity by reinforcing tight junctions;
- inhibiting the adhesion of UPEC strains to the mucosa of the urinary tract (activity mediated by oligomeric and polymeric PAC catabolites);

Furthermore, it has recently been found that PAC-A dimers and trimers are most responsible for the effect on the treatment and prevention of UTIs.

Preferably, the extract for use according to the present invention has a content in (weight in dimers + trimers of PAC-A) on (total PAC weight) between 18 and 26%.

A particularly preferred cranberry extract according to the present invention has the composition set forth in the following table 1 with respect to the relative degree of polymerization percentage.

**Table 1 - Cranberry extract PAC polymer composition**

| **Cranberry extract PAC** | **Relative degree of polymerization percentage** |
|---|---|
| Monomers | 11.50 |
| Dimers | 22.61 |
| Trim ers/tetramers | 25.97 |
| Pentamers/hexamers | 39.92 |
| Total | 100 |

In the specific example the ratio (weight of all the proanthocyanidin polymers) /(total weight of proanthocyanidins) is 88.5%

This extract is characterized in particular in that the PACs have the following composition in PAC-A and PAC-B given below in table 2.

**Table 2 - Total PAC-A and PAC-B composition**

| **Degree of polymerization** | **PAC-A %** | **PAC-B %** | **Ratio (PAC-A/PAC-B)** |
|---|---|---|---|
| Dimers | 1.83 | 1.60 | 1.14 |
| Trimers | 1.24 | 0.43 | 2.88 |
| Tetramers | 1.72 | 0.54 | 3.18 |
| Pentamers | 3.31 | 0.96 | 3.44 |
| Hexamers | 1.39 | 0.39 | 3.56 |
| **Total** | **9.49** | **3.92** | **2.42** |
| **Total relative %** | **70.77** | **29.23** | **2.4** |

Preferably, the PAC-A/PAC-B weight ratio is 2.42.

Preferably, the weight ratio (dimers + trimers of PAC-A) /(total PACs) is 22.89%.

The extract contained in the oral formulations object of the invention can be obtained by mixing appropriate weight ratios of commercially available extracts, the proanthocyanidin content of which has been analysed.

The extract for use object of the present invention is administered as an oral formulation containing it in combination with suitable excipients and/or diluents.

Preferably this oral formulation is a food supplement.

Preferably, the oral formulation contains at least 100 and more preferably between 200 and 400 mg of cranberry extract according to the present invention.

More preferably, the formulation also contains at least one active ingredient selected from vitamin C, D-mannose and green tea extract.

According to a particularly preferred solution, the oral formulation contains all three of the above active ingredients.

These oral formulations are for example in the form of tablets, controlled release tablets, pre-cut tablets, pre-cut controlled release tablets, capsules, controlled release capsules, sachets containing powder to be dissolved in water, Stick packs containing orosoluble powder, single-dose bottles, multi-dose bottles, candies, stick packs containing liquid to be swallowed.

An analysis which allowed different extracts to be characterized is shown below in example 1.

### EXAMPLE 1

There are two different methods for determining proanthocyanidin content:
- the Bates-Smith method associated with the method described in Pharmacopoeia, which is based on the depolymerization of PACs in an acidic environment. However, it is considered that such a method overestimates the results and does not allow the distinction between PAC-A and PAC-B;
- the colorimetric method BL-DMAC (Brunswick Laboratories 4-dimethylaminocnnamaldehyde) is based on an aldehyde condensation of the PACs.

The latter analysis is preferable to the previous one, as it is not subject to interference caused by other compounds contained in the cranberry (such as anthocyanins). In fact, the condensation reaction products absorb at 640 nm, unlike anthocyanins. On the other hand, the disadvantage of such a BL-DMAC method is the inability to distinguish PAC-As from PAC-Bs. Therefore, to overcome this disadvantage, an analytical system was developed based on the coupling between HPLC and mass spectrophotometry, or between HPLC and fluorescence (15).

Following the above method HPLC-FL-MS (FL, fluorescence and MS, mass spectrometry), the following was determined: 1) *the degree of polymerization* and 2) *the percentage content of PAC-A and PAC-B,* for the evaluation and comparison between different extracts.

### 1) Degree of polymerization

### 1.1 methods

For the purposes of the present invention, the raw materials were extracted in methanol with the aid of ultrasonic bath for 20 minutes. The samples were then centrifuged and placed in vials. The analyses were performed in HPLC-FLD. In particular, fluorescence analysis (or FLD) allows the quantification of total PACs (type A and type B) depending on the degree of polymerization.

The fluorescence analysis (FLD) allows the quantification of total type A
and type B PACs depending on the degree of polymerization (Table 3).

**Table 3 - retention times in relation to degree of polymerization**

| **Degree of polymerization** | **Retention times** |
|---|---|
| Monomers (Epicatechin-catechin) | 0-12 minutes |
| Dimers | 12-20 minutes |
| Trimers/tetramers | 20-30 minutes |
| Pentamers/hexamers | >30 minutes |

The analysis was performed using a Tosohas TSK gel amide 80.0mm ID x 15 cm L µ3.0 column as stationary phase and acetonitrile (A) and 1% formic acid water (B) according to the gradient shown in table 4.

**Table 4 - gradient %A and %B**

| Time | %A | %B | Flow (uL/min) |
|---|---|---|---|
| 0:00 | 90.0 | 10.0 | 200.0 |
| 20:00 | 80.0 | 20.0 | 200.0 |
| 25:00 | 80.0 | 20.0 | 200.0 |
| 45:00 | 35.0 | 65.0 | 200.0 |
| 51:00 | 35.0 | 65. | 200.0 |
| 52:00 | 90.0 | 10.0 | 200.0 |
| 60:00 | 90.0 | 10.0 | 200.0 |

A flow of 200 µL per minute was maintained.

An excitation wavelength of 231 nm and an emission wavelength of 321 nm was set for the fluorimeter.

A methanolic solution of PAC A2 dimer (Extrasynthese) at a concentration of 85-8.5 µg/ml was used as a reference standard.

### 1.2 Results

Tables 5a, 5b show the results regarding the percentage (% m/m) of the PACs, compared to the total extract content detected by HPLC-FLD, and tables 5c and 5d show the relative polymerization percentages.

**Table 5a**

| **Degree of Polymerization (%m/m)** | **EXTRACT 1** | **EXTRACT 2** | **EXTRACT 3** | **EXTRACT 4** |
|---|---|---|---|---|
| Monomers | 1.71±0.15 | 0.82±0.09 | 0.32±0.02 | 1.75±0.18 |
| Dimers | 5.47±0.27 | 0.98±0.05 | 0.22±0.04 | 2.31±0.04 |
| Trimers/tetramers | 9.50±0.16 | 0.90±0.06 | 0.24±0.00 | 2.72±0.08 |
| Pentamers/hexamers | 8.35±0.11 | 1.55±0.08 | 0.91±0.05 | 3.46±0.15 |
| **TOTAL (%m/m)** | **25.03** | **4.24** | **1.69** | **10.24** |

**Table 5b**

| **Degree of Polymerization (%m/m)** | **EXTRACT 5** | **EXTRACT 6** | **EXTRACT 7** | **EXTRACT 8** | **EXTRACT 9** |
|---|---|---|---|---|---|
| Monomers | 5.97±0.20 | 2.90±0.21 | 4.29±0.43 | 1.97±0.33 | 0.31±0.01 |
| Dimers | 2.17±0.67 | 2.15±0.12 | 2.99±0.23 | 2.65±0.37 | 0.18±0.01 |
| Trimers/tetramers | 4.52±0.71 | 4.26±0.02 | 6.20±0.48 | 3.56±0.82 | 0.23±0.01 |
| Pentamers/hexamers | 4.42±0.25 | 5.65±0.32 | 7.97±1.03 | 4.90±0.02 | 0.47±0.00 |
| **TOTAL (%m/m)** | **17.09** | **14.95** | **21.46** | **13.08** | **1.20** |

**Table 5c**

| **Degree of polymerization (% relative)** | **EXTRACT 1** | **EXTRACT 2** | **EXTRACT 3** | **EXTRACT 4** |
|---|---|---|---|---|
| Monomers | 6.82 | 19.31 | 19.05 | 17.09 |
| Dimers | 21.87 | 23.04 | 12.85 | 22.54 |
| Trimers/tetramers | 37.95 | 21.18 | 14.45 | 26.57 |
| Pentamers/hexamers | 33.36 | 36.48 | 53.90 | 33.81 |
| TOTAL (% relative) | 100 | 100 | 100 | 100 |

**Table 5d**

| **Degree of Polymerization (%m/m)** | **EXTRACT 5** | **EXTRACT 6** | **EXTRACT 7** | **EXTRACT 8** | **EXTRACT 9** |
|---|---|---|---|---|---|
| Monomers | 34.93 | 19.42 | 20.00 | 15.07 | 26.16 |
| Dimers | 12.72 | 14.37 | 13.93 | 20.27 | 14.70 |
| Trimers/tetramers | 26.46 | 28.46 | 28.91 | 27.21 | 19.49 |
| Pentamers/hexame | 25.87 | 37.76 | 37.16 | 37.45 | 39.65 |
| TOTAL (% relative) | 100 | 100 | 100 | 100 | 100 |

The extracts with the largest total PAC titre are 1 and 7. The extracts 5, 8, 6 and 4 follow. The extracts with the lowest PAC titre are 2, 3 and 9. From a composition point of view, considering the balance of the different degrees of polymerization of the flavan-3-ols, it can be observed that extract 3 has the highest relative amount of higher weight polymers (53% pentamers/hexamers) followed by extract 9 (40%), and in fact both provide a relatively high value with the pharmacopoeia method (which includes oxidative depolymerization -Reaction Bate Smith-) while the others are between 25-35% of high weight PACs as a percentage of the totals. It should be noted that extract 1 has the lowest content of monomeric flavan-3-ols compared to all the others and the highest content (in relative percentage but also as a weight amount) of trimers and dimers.

### 2) Percentage content of PAC-A and PAC-B

### 2.1 method

The calculation of the relative percentage of PAC-A and PAC-B for each commercial extract mentioned above can be derived by mass spectrometry (MS). The discrimination of PAC-A from PAC-B is based on the observation of deprotonated molecular ions [M-H]⁻.

Based on the literature data, a series of derivatives are selected which are characteristic of cranberry and are exemplified in table 6.

**Table 6**

| **Analytes** | **[M-H]-** | **number of A bonds** |
|---|---|---|
| PAC A dimers | 575 | 1 |
| PAC B dimers | 577 | 0 |
| PAC A trimers | 861 | 2 |
| | 863 | 1 |
| PAC B trimers | 865 | 0 |
| | 1147 | 3 |
| PAC A tetramers | 1149 | 2 |
| | 1151 | 1 |
| PAC B tetramers | 1153 | 0 |
| PAC A pentamers | 1433 | 4 |
| | 1435 | 3 |
| | 1437 | 2 |
| | 1439 | 1 |
| PAC B pentamers | 1441 | 0 |
| PAC A hexamers | 1727 | 1 |
| PAC B hexamers | 1729 | 0 |

For each extract analysed, the quantities of each PAC class are reported in order to compare the values and verify the prevalence of type "A" procyanidins. Quantities expressed as % by weight and percentages of PAC A and PAC B on the total PAC content of the extracts are given (Tables 7a-7g).

**Table 7a - EXTRACT 1**

| **Degree of polymerization** | **PAC-A %** | **PAC-B %** | **A/B Ratio** |
|---|---|---|---|
| Dimers | 4.45 | 1.02 | 4.4 |
| Trimers | 2.35 | 1.23 | 1.9 |
| Tetramers | 3.96 | 1.96 | 2 |
| Pentamers | 4.97 | 1.16 | 4.3 |
| Hexamers | 1.74 | 0.48 | 3.6 |
| **Total** | 17.46 | 5.86 | **2.9** |
| **Relative % of PAC A or B on TOTAL PAC** | **74.87** | **25.13** | |

**Table 7b - EXTRACT 2**

| **Degree of polymerization** | **PAC-A** | **PAC-B %** | **A/B Ratio** |
|---|---|---|---|
| Dimers | 0.30 | 0.68 | 0.44 |
| Trimers | 0.15 | 0.33 | 0.44 |
| Tetramers | 0.09 | 0.33 | 0.27 |
| Pentamers | 0.39 | 0.55 | 0.70 |
| Hexamers | 0.31 | 0.30 | 1.0 |
| **Total** | **1.24** | **2.19** | **0.56** |
| **Relative % of PAC A or B on TOTAL PAC** | **36.15** | **63.85** | |

**Table 7c - EXTRACT 4**

| **Degree of polymerization** | **PAC-A %** | **PAC-B %** | **A/B Ratio** |
|---|---|---|---|
| Dimers | 0.85 | 1.46 | 0.58 |
| Trimers | 0.41 | 1.02 | 0.4 |
| Tetramers | 0.25 | 1.05 | 0.2 |
| Pentamers | 0.87 | 1.23 | 0.7 |
| Hexamers | 0.70 | 0.66 | 1.1 |
| **Total** | **3.08** | **5.41** | **0.56** |
| **Relative % of PAC A or B on TOTAL PAC** | **36.22** | **63.78** | |

**Table 7d - EXTRACT 5**

| **Degree of polymerization** | **PAC-A %** | **PAC-B %** | **A/B Ratio** |
|---|---|---|---|
| Dimers | 0.54 | 1.63 | 0.33 |
| Trimers | 1.26 | 0.96 | 1.25 |
| Tetramers | 1.16 | 1.14 | 1 |
| Pentamers | 2.24 | 0.83 | 2.7 |
| Hexamers | 0.82 | 0.52 | 1.57 |
| **Total** | 6.03 | 5.08 | 1.1 |
| **Relative % of PAC A or B on TOTAL PAC** | **54.24** | **45.76** | |

**Table 7e - EXTRACT 6**

| **Degree of polymerization** | **PAC-A %** | **PAC-B %** | **A/B Ratio** |
|---|---|---|---|
| Dimers | 0.82 | 1.33 | 0.6 |
| Trimers | 1.24 | 0.90 | 1.37 |
| Tetramers | 0.83 | 1.30 | 0.6 |
| Pentamers | 1.21 | 2.00 | 0.61 |
| Hexamers | 1.39 | 1.04 | 1.33 |
| **Total** | 5.48 | 6.58 | 0.83 |
| **Relative % of PAC A or B on TOTAL PAC** | **45.48** | **54.52** | |

**Table 7f - EXTRACT 7**

| **Degree of polymerization** | **PAC-A %** | **PAC-B %** | **A/B Ratio** |
|---|---|---|---|
| Dimers | 1.35 | 1.64 | 0.82 |
| Trimers | 1.87 | 1.56 | 1.2 |
| Tetramers | 1.10 | 1.67 | 0.65 |
| Pentamers | 2.35 | 2.17 | 1.1 |
| Hexamers | 2.09 | 1.36 | 1.53 |
| **Total** | 8.76 | 8.40 | 1.04 |
| **Relative % of PAC A or B on TOTAL PAC** | **51.06** | **48.94** | |

**Table 7g - EXTRACT 8**

| **Degree of polymerization** | **PAC-A %** | **PAC-B %** | **A/B Ratio** |
|---|---|---|---|
| Dimers | 1.17 | 1.48 | 0.79 |
| Trimers | 0.88 | 0.88 | 1.0 |
| Tetramers | 0.77 | 1.03 | 0.75 |
| Pentamers | 1.42 | 1.26 | 1.12 |
| Hexamers | 1.30 | 0.92 | 1.4 |
| **Total** | 5.54 | 5.57 | 0.99 |
| **Relative % of PAC A or B on TOTAL PAC** | **49.88** | **50.12** | |

For extracts 3 and 9, under the preparation conditions of the other compounds, the mass analysis is found to provide excessively low signals, due to the low amounts of PAC present. This measurement is excluded for the moment.

The results show that for the analysed samples, the extracts 1, 5 and 7 have a greater percentage of PAC-A than PAC-B.

The tables with the comparison and the ratios between PAC A/PACB for each category of analytes considered allow to make considerations on the different extracts from a composition point of view. Extract 1 has the highest ratios, but this is expected because it is an extract which undergoes a partial purification process precisely to increase the PAC-A content.

Based on this, the Applicant chose to mix extracts 1 and 8, in weight ratios 75:25. An example of cranberry extract whose features are given in tables 1 and 2 above particularly for use according to the present invention is derived.

### REFERENCES

*1.* P. P. B. Bientinesi, «Le infezioni delle vie urinarie, » 2014*.*
2. N. Foschi, R. Bientinesi, G. Palermo, F. Pinto e al, «Le infezioni delle vie urinarie», Urologia, vol. 81, n. 25, pp. 16-25, 2014*.*
*3.* W. C. H. Flores-Mireles, «Urinary tract infections: epidemiology, mechanisms of infection,» 2015*.*
*4.* B. e. Mobley, «Preventing urinary tract infection: progress toward an effective Escherichia coli vaccine,» 2012*.*
5. C. C. K.-E. H. M. O. S. S.-R. V. Blumberg, «Cranberry and their bioactive constituents in human health,» 2013*.*
*6.* L. Domenici, M. Monti, C. Bracchi, M. Giorgini e al, «D-mannose: a promising support for acute urinary tract infections in women. A pilot study,» Eur Rev Med Pharmacol Sci, vol. 20, pp. 2920-2925, 2016*.*
7. G. I. Capasso, Fitoterapia, 2006*.*
8. M. Maffei, «Sinergia fra Oximacro (estratto di mirtillo rosso, Vaccinium macrocarpon o cranberry), D-mannosio e Ononis spinosa (Ononide) per la riduzione delle infezioni del tratto urinario,» Ginecorama, n. 2, 2017*.*
*9.* Cicero, Tratto italiano di nutraceutica clinica, 2017*.*
*10.* A. Occhipinti, A. Germano e M. Maffei, «Prevention of urinary tract infection with Oximacro, a cranberry extract with a high content of A-type proanthocyanidins: a pre-clinical double-blind controlled study, » Urology J, vol. 13, n. 2, pp. 2640-2649, 2016*.*
11. G. Peron, S. Sut, A. Pellizzaro e al, «The antiadhesive activity of cranberry phytocomplex studied by metabolomics: Intestinal PAC-A metabolites but not intact PAC-A are identified as markers in active urines against uropathogenic Escherichia Coli, » Fitot*.*
*12.* B. C. B.-P. G. M. T. S. L. Howell, «Dosage effect on uropathogenic Escherichia coli anti-adhesion activity in urine following consumption of cranberry powder standardized for proanthocyanidin content: a multicentric randomized double blind study.,» 2010*.*
*13.* B. C. B. S. Lavigne, « In-vitro and in-vivo evidence of dose-dependent decrease of uropathogenic Escherichia coli virulence after consumption of commercial Vaccinium macrocarpon (cranberry) capsules.,» 2008*.*
14. ANSES, «Opinion of the French Agency for Food, Environmental and Occupational Health & Safety on the assessment of the potential effects of cranberry on community-acquired urinary tract infections,» 2010*.*
15. M. Maffei, «Vaccinium macrocarpon: caratterizzazione e quantificazione delle proantocianidine negli estratti di cranberry,» Natural, pp. 26-33, 2016*.*
16. G. I. B. Ermel, «Inhibition of adhesion of uropathgenic Escherichia coli Bacteria to Uroepithelial cells by extracts from cranberry,» 2011*.*
17. P. Z. K. G. Ou, «Transport of Cranberry A-type Procyanidin Dimers, Trimers and Tetramers across Monolayers of Human Intestinal Epithelial Cao-2 Cells,» 2012*.*
18. M. H. T. S. Deprez, «Transport of Proanthocyanidin dimer, Trimer, and Polymer Across Monolayers of human Intestinal Epithelial Caco-2 Cells,» 2001*..*
19. S. F. Howell, «Comparison of the Anti-Adhesion Activity of Three Different Cranberry Extracts on Uropathogenic P-fimbriated Escherichia coli: a Randomized, Double-blind, Placebo Controlled, Ex Vivo, Acute Study.,» 2010*.*
20. E.F., S.P., M.A., M.A., B. De Llano, «Anti-Adhesive Activity of cranberry phenolic Compounds and theirr microbial-derived metabolites against uropathogenic Escherichia coli in bladder epithelial cell cultures,» 2015*.*
21. S. P. B. V. C. D. Peron, «The antiadhesive activity of cranberry phytocomplex studied by metabolomics: Intestinal PAC-A metabolites but not intact PAC-A are identified as markers in active urines against uropathogenic Escherichia coli,» 2017*.*
22. B. R. P. M. L. S. Deprez, «Polymeric Proanthocyanidins are catabolized by human colonic microflora into low-molecular phenolic acids,» 2000*.*
23. (s.d.). M. G. R. A. Bazzocco, «Factors affecting the conversion of apple polyphenols to phenolic acids and fruit matrix to short-chain fatty acids by human faecal microbiota in vitro.,» 2008*.*
24. M. S. M. K. R. Feliciano, «Supercritical fluid extraction (SFE) of cranberries does not extract oligomeric proanthocyanidins (PAC) but does alter the chromatography and bioactivity of PAC fractions extracted from SFE residues.,» 2015*.*
25. H. F. S. K. R. K. Pierre, «Cranberry Proanthocyanidins improve intestinal sIgA during elemental enteral nutrition, » 2014*.*
26. H. D. K. G. F. J. Holderness, «Response of γδ T cells to plant-derived tannins,» 2008*.*
27. G. N. Bitzer, «Cocoa Procyanidins with Different Degrees of Polymerization Possess Distinct Activities in Models of Colonic Inflammation,» 2015*.*
28. H.L., G.D., G.a. G. B. K. K. C. R. A., L. Peron, «Exploring the Molecular Pathways Behind the Effects of Nutrients and Dietary Polyphenols on Gut Microbiota and Intestinal Permeability: A Perspective on the Potential of Metabolomics and Future Clinical Ap*.*

## Claims

1. Cranberry extract wherein the degree of polymerization (% m/m) in total proanthocyanidins (PACs) is between 12.5 and 15%, preferably between 13 and 14.5%, and wherein the ratio (total proanthocyanidin polymers weight)/(proanthocyanidins total weight) is > 60%, preferably between 70 and 90%,
for use as a sole active ingredient or as an adjuvant in the treatment and prevention of uncomplicated lower urinary tract infections.

2. Extract for use according to claim 1, wherein the total proanthocyanidins consist of:
• type A proanthocyanidins (PACs-A), condensed among each other through the C2-O-C5 or C2-O-C7 bond, and
• type B proanthocyanidins (PACs-B), condensed together through the C4-C6 or C4-C8 bond;
and wherein the PAC-A/PAC-B weight ratio is comprised between 1.5 and 3, preferably between 1.8 and 2.6.

3. Extract for use according to claim 1 or 2, wherein the weight ratio (PACs-A dimers + trimers)/(total PACs) is between 18 and 25%.

4. Extract for use according to any one of claims from 1 to 3, wherein said extract is administered in the form of an oral formulation which contains it in combination with suitable excipients and/or diluents.

5. Extract for use according to claim 4, wherein said oral formulation is a food supplement.

6. Extract for use according to claim 4 or 5, wherein said oral formulation contains at least 100 mg, preferably between 200 and 400 mg of Cranberry extract.

7. Extract for use according to any one of claims from 4 to 6, wherein said oral formulation further comprises at least one active ingredient selected from vitamin C, D-mannose and green tea.

8. Extract for use according to claim 7, containing vitamin C, D-mannose and green tea.
